# EUROPÄISCHE PATENTANMELDUNG

(11) **EP 1 366 740 A1**
(43) Veröffentlichungstag der Anmeldung: **03.12.2003**
(21) Anmeldenummer: 03011975.4
(22) Anmeldetag: 28.05.2003
(51) Int. Cl.: A61K 7/00, A61K 7/06, A61K 7/075, A61K 7/08

(54) **Getrübtes Pflegeshampoo**

(30) Priorität: 31.05.2002 DE 10224024
(71) Anmelder: Beiersdorf AG, 20245 Hamburg (DE)
(72) Erfinder: Demitz, Michael, 22529 Hamburg (DE); Argembeaux, Horst, 21465 Wentorf (DE); Albrecht, Harald, 22083 Hamburg (DE); Heitmann, Birgit, 22769 Hamburg (DE); Primmel, Bettina, 20146 Hamburg (DE); Kaiser, Elke, 22769 Hamburg (DE)

(57) **Zusammenfassung**

Kosmetische Zubereitung einhaltend
a) ein oder mehrere Guar-Hydroxypropyltrimethylammoniumchloride,
b) ein oder mehrere Trübungsmittel,
c) ein oder mehrere Tenside,
neben gegebenenfalls weiteren kosmetischen Wirk-, Hilfs-, und Zusatzstoffen.

## Beschreibung

Die vorliegende Erfindung betrifft eine kosmetische Zubereitung mit Trübungsmittel und ihre Verwendung.

Bei der Körperpflege des Menschen spielt die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurück ließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten um den einzelnen Anforderungen an das Produkt gerecht zu werden:

Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampoo-Tensid ist beispielsweise Alkylethersulfat.

Darüber hinaus enthalten Shampoos eine Reihe von Konsistenzregulatoren, die der Zubereitung die gewünschte Viskosität verleihen. Diese Verdicker bewirken eine Vergrößerung der Tensidmicellen bzw. eine Quellung der Wasserphase der Zubereitung. Verdicker können aus chemisch sehr unterschiedlichen Stoffklassen gewählt werden. So werden u.a. Elektrolyte (z.B. Natriumchlorid), Alkanolamide (z.B. Fettsäure-Monoethanolamide), niedrig ethoxylierte Fettalkohole (z.B. Diethylenglycolmonolaurylether), hochethoxylierte Ether, Ester und Diester sowie polymere Verdicker eingesetzt. Zu den polymeren Verdickern zählen beispielsweise Celluloseether. Darüber hinaus finden auch Polyacrylate und Hydrokolloide als Verdicker Verwendung. Polymere Verdicker haben den großen Vorteil, dass die durch sie erzeugte Viskosität weitgehend temperaturunabhängig ist.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen. Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen, in der Regel kationische Polymere, stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.

Ein Nachteil am Stande der Technik besteht in dem Umstand, dass durch häufiges Waschen oder chemische Behandlung (Bleichen, Färben) das Haar stark geschädigt wird. Insbesondere führt die Entfernung/Zerstörung von natürlich im Haar vorkommenden Fettkomponenten zu einem Verlust an Glätte, Geschmeidigkeit und Glanz. Das Haar erscheint dann trocken und spröde. Um diese Schäden ausgleichen zu können, werden den Haarpflegeprodukten in der Regel große Mengen an kationischen Polymeren als Haarkonditionierern zugesetzt. Um den Fettverlust auszugleichen, werden derartigen Produkten häufig Silikonverbindungen oder weitere hydrophobe Konditionierer zugesetzt. Diese nach dem Stande der Technik hergestellten Produkte haben jedoch eine Reihe von Nachteilen:
■ Es lagern sich bei jeder Anwendung herkömlicher Produkte zusätzliche Schichten an Konditionierer auf dem Haar ab (engl. build-up-effect). Das Haar wird durch die großen Mengen an Konditionierern beschwert und bekommt ein strähniges Aussehen.
■ Die Produkte enthalten wasserlösliche und fettlösliche Bestandteile, die zu einer Emulsion dispergiert sind. Die Herstellung solcher Emulsionen ist jedoch sehr aufwendig. Es kann dabei immer wieder zu Unverträglichkeiten von einzelnen Inhaltsstorfen (Aufrahmen der Fettphase, Ausfällungen) kommen.

Es war daher die Aufgabe der vorliegenden Erfindung die Mängel des Standes der Technik zu beseitigen oder zumindest zu lindern und gut verträgliche Haarpflegeprodukte zu entwickeln, welche selbst stark geschädigtem Haar wieder zu neuem Glanz, Fülle und Spannkraft verhelfen. Um die Probleme bei der Herstellung von Emulsionen zu umgehen, sollte die Zubereitung auf einer wässrigen Phase basieren. Da beim Verbraucher jedoch ein Pflegeeffekt insbesondere mit einem emulsionsartigen, cremigen Erscheinungsbild einer Formulierung in Verbindung gebracht wird, sollte die Zubereitung äußerlich den Eindruck einer Emulsion erwecken.

Überraschend gelöst wird die Aufgabe durch eine kosmetische Zubereitung einhaltend
a) ein oder mehrere Guar-Hydroxypropyltrimethylammoniumchloride,
b) ein oder mehrere Trübungsmittel,
c) ein oder mehrere Tenside,
neben gegebenenfalls weiteren kosmetischen Wirk-, Hilfs-, und Zusatzstoffen.

Das äußere Erscheinungsbild der erfindungsgemäßen Zubereitungen entspricht dem einer Emulsion: Sie ist milchig-trübe im Aussehen und von cremiger Sensorik beim Auftragen auf die Haut bzw. das Haar. Die Pflegeeigenschaften entsprechen denen von ölhaltigen, emulsionsartigen Formulierungen. Wird das Haar mit der erfindungsgemäßen Zubereitung behandelt, so läßt sich ein deutlicher Pflegeeffekt beobachten. Die Frisur bekommt selbst bei stark geschädigtem Haar wieder Glanz, Fülle und Volumen, ohne dass bei wiederholter Anwendung der gefürchtete "build-up"-Effekt in Erscheinung tritt.

Es ist erfindungsgemäß von Vorteil, wenn die erfindungsgemäßen Guar-Hydroxypropyltrimethylammoniumchloride eine Ladungsdichte von 0,4 bis 1,0 meq/g, sowie ein Molekulargewicht von 600000 bis 2000000 aufweisen. Erfindungsgemäß besonders bevorzugt ist das Guar-Hydroxypropyltrimethylammoniumchlorid (Cosmedia Guar C 261 der Firma Cognis).

Erfindungsgemäß vorteilhaft werden ein oder mehrere Guar-Hydroxypropyltrimethylammoniumchloride in einer Konzentration von 0,01 bis 3 Gewichts-%, bevorzugt in einer Konzentration von 0,05 bis 2,5 Gewichts-% und ganz besonders bevorzugt in einer Konzentration von 0,08 bis 2 Gewichts-%, jeweils bezogen auf das Gesamtgewicht der Zubereitung, eingesetzt.

Erfindungsgemäß versteht man unter Trübungsmittel kolloid trüblösliche Hilfsstoffe, die insbesondere transparenten kosmetischen Zubereitungen zum Zwecke der Herstellung eingetrübter Zubereitungen oder zur Eintrübung der Zubereitung selbst zugesetzt werden.

Das oder die erfindungsgemäßen Trübungsmittel können aus einem Inhaltsstoff oder aus einer Mischung von mehreren Verbindungen bestehen. Beide Formen werden in der vorliegenden Erfindung der Einfachheit halber unter dem begriff Trübungsmittelzubereitung oder Trübungsmittel zusammengefasst. Erfindungsgemäß vorteilhafte Trübungsmittelzubereitungen können beispielsweise aus der folgenden Auflistung gewählt werden:
■ PEG-3 Distearat (z.B. CUTINA TS der Firma Cognis),
■ eine Kombination aus Glycoldistearat, Glycerin, Laureth-4 und Cocamidopropylbetain (z.B. Euperlan PK 3000 und Euperlan PK 4000 der Firma Cognis),
■ eine Kombination aus Glycoldistearat, Cocosglucosiden, Glyceryloleat und Glycerylstearat (z.B. Lamesoft TM Benz der Firma Cognis)
■ Styrol/Acrylat Copolymere (z.B. Acusol OP 301 von Rohm & Haas)

Es ist erfindungsgemäß von Vorteil, wenn ein als Trübungsmittel PEG-3 Distearat (z.B. CUTINA TS der Firma Cognis) und/oder eine Kombination aus Glycoldistearat, Cocosglucosiden, Glyceryloleat und Glycerylstearat (z.B. Lamesoft TM Benz der Firma Cognis), eingesetzt werden.
Dabei soll diese Auflistung selbstverständlich in keinster Weise limitierend sein. Vielmehr sind grundsätzlich alle in der Kosmetik einsetzbaren Trübungsmittel erfindungsgemäß vorteilhaft einsetzbar.

Es ist erfindungsgemäß von Vorteil, wenn ein oder mehrere Trübungsmittel in einer Konzentration von 0,1 bis 5,0 Gewichts-%, bevorzugt in einer Konzentration von 0,2 bis 4,5 Gewichts-% und ganz besonders bevorzugt in einer Konzentration von 0,3 bis 4,0 Gewichts-%, jeweils bezogen auf das Gesamtgewicht der Zubereitung eingesetzt werden.
Bei Trübungsmittelzubereitungen welche aus mehreren Komponenten bestehen, beziehen sich dabei die Konzentrationsangaben auf die Trübungsmittelzubereitungsmischung.

Es ist Ferner erfindungsgemäß besonders vorteilhaft bei den Mischungen aus Trübungsmittel und erfindungsgemäßem Guar-Hydroxypropyltrimethylammoniumchlorid die folgenden Konzentrationsverhältnisse zu beachten:
Bei einer Mischung aus Trübungsmittel mit Guar-Hydroxypropyltrimethylammoniumchlorid, ist es vorteilhaft auf 1 Gewichtsanteil Trübungsmittel, von 0,01 bis 1 Gewichtsanteile Guar-Hydroxypropyltrimethylammoniumchlorid einzusetzen, besonders vorteilhaft ist es auf auf 1 Gewichtsanteil Trübungsmittel, von 0,05 bis 1 Gewichtsanteile Guar-Hydroxypropyltrimethylammoniumchlorid einzusetzen.

Die erfindungsgemäßen kosmetischen Zubereitungen enthalten Tenside. Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroyisarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole und
■ ethoxylierte Amine
und insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders bevorzugt, wenn als Tenside anionische, amphotere und/oder nichtionische Tenside eingesetzt werden wobei es ganz besonders bevorzugt ist, wenn als Tenside Natriumlaurylethersulfat, Cocoamidopropylbetain und/oder Dinatrium PEG-5 Laurylcitratsulfosuccinat und/oder N-Kokosfettsäureamidoethyl-N-hydroxyethylglycinat Natriumsalze eingesetzt werden.

Ferner können Polysorbate als waschaktive Agentien erfindungsgemäß vorteilhaft in die Emulsion eingearbeitet werden.

Es ist erfindungsgemäß von Vorteil, wenn ein oder mehrere Tenside in einer Konzentration von 1 bis 25 Gewichts-%, bevorzugt in einer Konzentration 7 von 20 bis Gewichts-% und ganz besonders bevorzugt in einer Konzentration von 8 bis 18 Gewichts-%, jeweils bezogen auf das Gesamtgewicht der Zubereitung eingesetzt wird.

Ferner ist es erfindungsgemäß von Vorteil, wenn in den erfindungsgemäßen Zubereitungen Salze eingesetzt werden. Diese führen zu einer Verdickung der Zubereitung, so dass Zubereitungen mit Viskositäten von 2000 mPas bis 6000 mPas hergestellt werden können. Unter Salzen sind ein- oder mehrwerteige Alkalimetallverbindungen bzw. Erdalkalimetallverbindungen mit Halogenanionen zu verstehen. Besonders bevorzugt wird Natriumchlorid eingesetzt.

Es ist erfindungsgemäß von Vorteil ein oder mehrere Alkylisalze bzw. Erdalkylisalze in einer Konzentration von 0,1 bis 3 Gewichts-%, bezogen auf das Gesamtgewicht der Zubereitung einzusetzen.

Ferner ist es erfindungsgemäß von Vorteil, wenn in den erfindungsgemäßen Zubereitungen Verdicker eingesetzt werden. Diese können beispielsweise vorteilhaft aus weiteren Verbindungen der Gruppe der Gummen gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate als erfindungsgemäß vorteilhafte Verdicker.

Unter den Cellulosederivaten befinden sich z.B. Methylcellulose, Carboxymethylcellulose, Hydroxyethylcellulose, Hydroxypropylmethylcellulose als erfindungsgemäß vorteilhafte Verdicker.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form als Verdicker verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Noveon (Carbopol 980, 981, 1382, 5984, 2984, ETD 2001, ETD 2020, ETD 2050 oder Pemulen TR1 & TR2).

Unter den Polymeren befinden sich z.B. Polyacrylamide (Seppigel 305), Polyvinylaikohole, PVP, PVP / VA Copolymere, Polyglycole.

Es ist erfindungsgemäß von Vorteil ein oder mehrere Verdicker in einer Konzentration von 0,1 bis 5 Gewichts-%, bezogen auf das Gesamtgewicht der Zubereitung einzusetzen.

Vorteilhaft, wenn auch nicht zwingend, können die erfindungsgemäßen Zubereitungen Konservierungsmittel enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ^{TM} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Die wässrige Phase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Ultrez 10, jeweils einzeln oder in Kombination.

Die kosmetischen Zubereitungen gemäß der Erfindung können kosmetische Hilfsstoffe enthalten, wie sie üblicherweise in solchen Zubereitungen verwendet werden, z. B.

Konservierungshelfer, Komplexbildner, Antioxidantien, Puffer, Lösungsvermittler, Dispergiermittel, Bakterizide, Parfüme, Substanzen zum Verhindem oder Steigern des Schäumens, Farbstoffe, Pigmente, die eine färbende Wirkung haben, Verdickungsmittel, Perlglanzpigmente, weichmachende, anfeuchtende und/oder feuchhaltende Substanzen, Fette, Öle, Wachse oder andere übliche Bestandteile einer kosmetischen oder dermatologischen Formulierung wie Emulgatoren, Alkohole, Polyole, Polymere, Schaumstabilisatoren, Elektrolyte, organische Lösungsmittel oder Silikonderivate.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Antischuppenwirkstoffe, Pflanzenextrakte, Vitamine, Wirkstoffe.

Insbesondere ist es erfindungsgemäß von Vorteil der erfindungsgemäßen Zubereitung Vitamine, Pflanzenextrakte und UV-Lichtschutzfilter zuzusetzen. So ist beispielsweise der Zusatz von Calcium-Vitamin-Komplexen erfindungsgemäß besonders vorteilhaft.

Ferner ist es im Sinne der vorliegenden Erfindung, der erfindungsgemäßen Zubereitung Perlglanzpigmente, Glimmer und/oder Effektpigmente zuzusetzen, um die Zubereitung optisch attraktiver zu gestalten.

Die erfindungsgemäßen Zubereitungen stellen in jeglicher Hinsicht überaus befriedigende Präparate dar, welche nicht auf die in dieser Schrift erwähnte Rohstoffauswahl begrenzt sind.

Es ist erfindungsgemäß vorteilhaft, wenn die erfindungsgemäße kosmetische Zubereitung in einer Flasche, Quetschflasche, Pumpspray-, oder Aerosoldose aufbewahrt und aus dieser heraus angewendet werden. Entsprechend sind auch Flaschen, Quetschflaschen, Pumpspray-, oder Aerosoldosen, welche eine erfindungsgemäße Zubereitung enthalten, erfindungsgemäß.

Die erfindungsgemäßen Zubereitungen werden vorteilhaft zur Pflege des Haars, insbesondere des Kopfhaares, eingesetzt.

Erfindungsgemäß ist die Verwendung der erfindungsgemäßen kosmetischen Zubereitung als Haarshampoo sowie die Verwendung zum Konditionieren des Haares.

Die folgenden Beispiele, in welchen Waschpräparate zur Haarpflege beschrieben werden, sollen die erfindungsgemäßen Zusammensetzungen erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

Beispielrezepturen

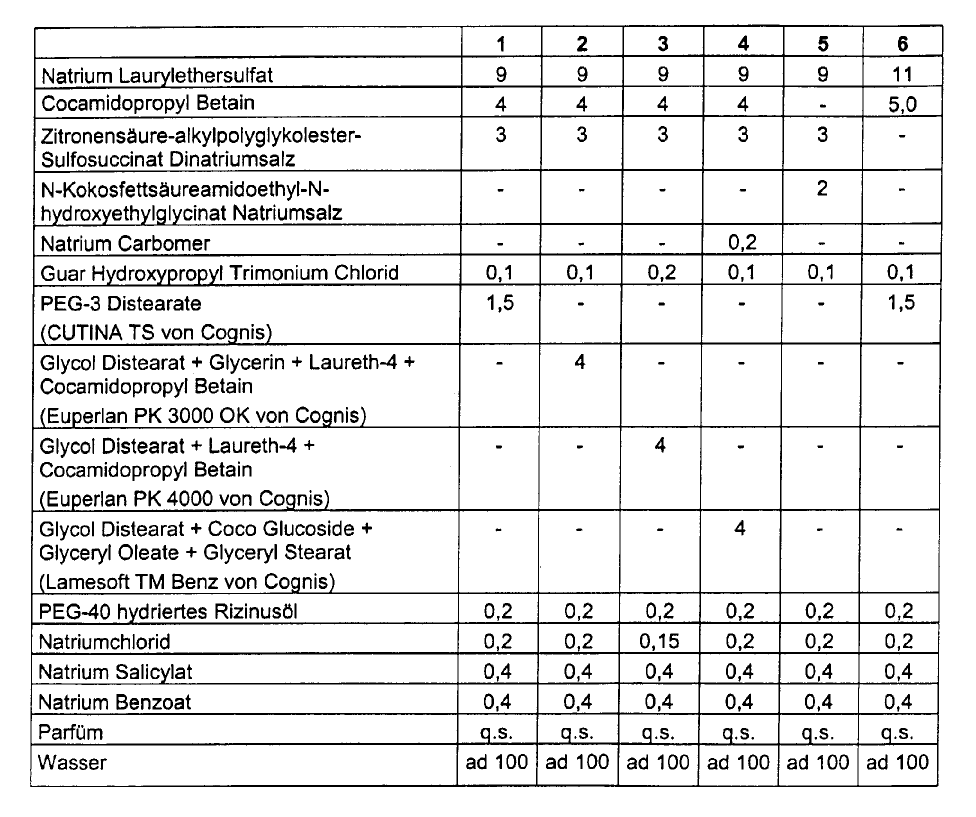

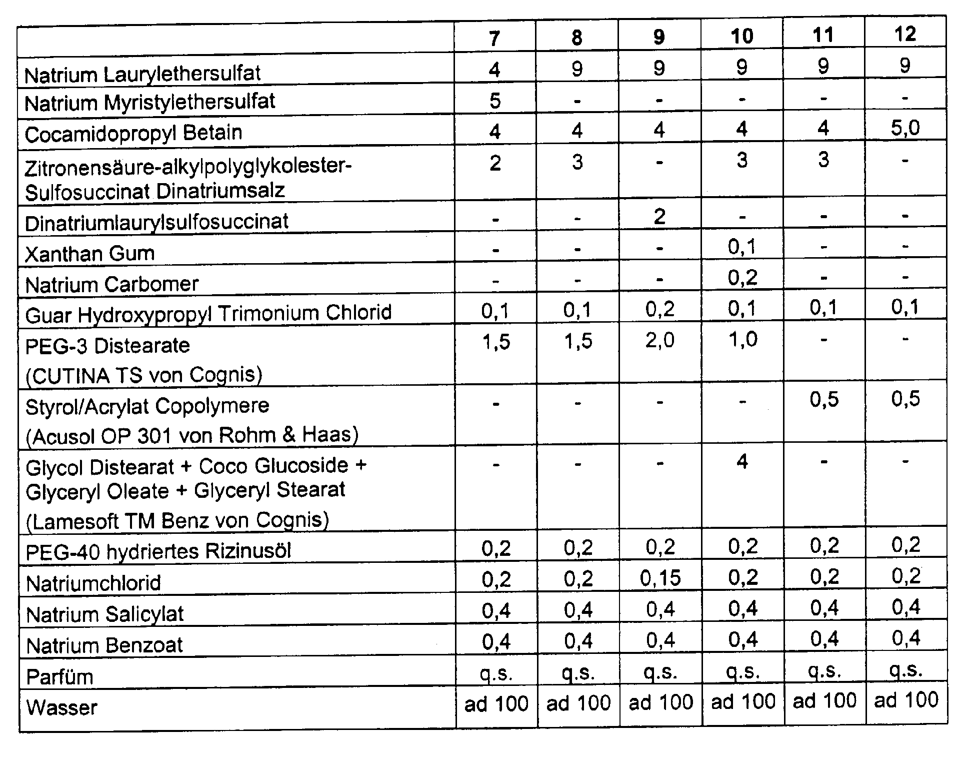

## Patentansprüche

1. Kosmetische Zubereitung einhaltend
a) ein oder mehrere Guar-Hydroxypropyltrimethylammoniumchloride,
b) ein oder mehrere Trübungsmittel,
c) ein oder mehrere Tenside, neben gegebenenfalls weiteren kosmetischen Wirk-, Hilfs-, und Zusatzstoffen.

2. Kosmetische Zubereitung nach Anspruch 1, **dadurch gekennzeichnet, dass** das oder die Guar-Hydroxypropyltrimethylammoniumchloride eine Ladungsdichte von 0,4 bis 1,0 meq/g, sowie ein Molekulargewicht von 600000 bis 2000000, aufweisen.

3. Kosmetische Zubereitung nach einem der Ansprüche 1 oder 2, **dadurch gekennzeichnet, dass** das oder die Trübungsmittel gewählt werden aus den Trübungsmittelzubereitungen:
■ PEG-3 Distearat,
■ eine Kombination aus Glycoldistearat, Glycerin, Laureth-4 und Cocamidopropylbetain,
■ eine Kombination aus Glycoldistearat, Cocosglucosiden, Glyceryloleat und Glycerylstearat
■ Styrol/Acrylat Copolymere

4. Kosmetische Zubereitung nach einem der Ansprüche 1 bis 4, **dadurch gekennzeichnet, dass** als Tenside anionische, amphotere und/oder nichtionische Tenside und ganz besonders bevorzugt, Natriumlaurylethersulfat, Cocoamidopropylbetain und/oder Zitronensäure-alkylpolyglykolester-Sulfosuccinat Dinatriumsalze und/oder N-Kokosfettsäureamidoethyl-N-hydroxyethylglycinat Natriumsalze eingesetzt werden.

5. Kosmetische Zubereitung nach einem der Ansprüche 1 bis 4, **dadurch gekennzeichnet, dass** sie
a) ein oder mehrere mehrere Guar-Hydroxypropyltrimethylammoniumchloride in einer Konzentration von 0,01 bis 3,0 Gewichts-%,
b) ein oder mehrere Trübungsmittel in einer Konzentration von 0,1 bis 5 Gewichts-%,
c) ein oder mehrere Tenside in einer Konzentration von 1 bis 25 Gewichts-% jeweils bezogen auf das Gesamtgewicht der Zubereitung enthalten.

6. Kosmetische Zubereitung nach einem der Ansprüche 1 bis 5, **dadurch gekennzeichnet, dass** die Zubereitung Verdicker enthält.

7. Verwendung einer kosmetischen Zubereitung nach einem der vorhergehenden Ansprüche als Haarshampoo.

8. Verwendung einer kosmetischen Zubereitung nach einem der vorhergehenden Ansprüche zum Konditionieren des Haares.
